# EUROPÄISCHE PATENTANMELDUNG

(11) **EP 3 053 521 A1**
(43) Veröffentlichungstag der Anmeldung: **10.08.2016**
(21) Anmeldenummer: 16152748.6
(22) Anmeldetag: 26.01.2016
(51) Int. Cl.: A61B 5/097, A61M 15/00

(54) **MUNDSTÜCK FÜR EINE VORRICHTUNG ZUR MESSUNG EINES PARAMETERS VON ATEMLUFT UND ATEMLUFTMESSGERÄT**

(30) Priorität: 03.02.2015 DE 102015201826
(71) Anmelder: ROBERT BOSCH GMBH, 70442 Stuttgart (DE)
(72) Erfinder: Reisinger, Daniel, 72108 Rottenburg-Wendelsheim (DE); Mueller, Klaus, 70449 Stuttgart (DE); Richter, Torsten, 64285 Darmstadt (DE)

(57) **Zusammenfassung**

Die Erfindung betrifft ein Mundstück (104) für eine Vorrichtung zur Messung eines Parameters von Atemluft, wobei eine Endkante (200) einer Wandung (116) des Mundstücks (104) eine Öffnung (202) zum Einblasen von Atemluft in das Mundstück (104) begrenzt, wobei sich eine Öffnungsfläche (213) der Öffnung (202) quer zu einer Strömungsachse (114) von in das Mundstück (104) eingeblasener Atemluft erstreckt. Eine Kontur der Öffnung (202) bildet in Richtung der Strömungsachse gesehen eine D-Form oder eine Linsenform aus und die Öffnung (202) ist durch einen ersten Öffnungsabschnitt (206) und einen an den ersten Öffnungsabschnitt (206) angrenzenden zweiten Öffnungsabschnitt (208) begrenzt.

## Beschreibung

### Stand der Technik

Die vorliegende Erfindung bezieht sich auf ein Mundstück für eine Vorrichtung zur Messung eines Parameters von Atemluft sowie ein Atemluftmessgerät und Steuergerät für ein Computerspiel.

Für die Messungen der Lungenfunktion oder der Gaszusammensetzung des Atemgases werden Masken oder Mundstücke verwendet. Diese Verbrauchsartikel werden benötigt, damit die Messgeräte nicht durch Keime kontaminiert werden. Manche Verbrauchsartikel enthalten weitere Funktionen. Während Masken über Mund und/oder Nase gestülpt werden, werden die Mundstücke von den Lippen umschlossen. Derzeit am Markt verfügbare Mundstücke haben im Querschnitt entweder eine runde oder eine ovale Form. Zu weites Einführen des Mundstücks in den Mund wird durch eine haptische Rückmeldung an den Lippen über umlaufende Erhöhungen oder durch Riffelungen erreicht.

### Offenbarung der Erfindung

Vor diesem Hintergrund werden mit dem hier vorgestellten Ansatz ein Mundstück für eine Vorrichtung zur Messung eines Parameters von Atemluft sowie ein Atemluftmessgerät gemäß den Hauptansprüchen vorgestellt. Vorteilhafte Ausgestaltungen ergeben sich aus den jeweiligen Unteransprüchen und der nachfolgenden Beschreibung.

Ein Mundstück für eine Vorrichtung zur Messung eines Parameters von Atemluft mit einem D-förmigen oder linsenförmigen Öffnungsquerschnitt einer Öffnung zum Einblasen der Atemluft in das Mundstück ermöglicht einen einfach zu erreichenden Lippenschluss um das Mundstück, da die hierin vorgeschlagene Form des Mundstücks an einem Kontaktbereich der Lippen mit dem Mundstück der Lippenform im entspannten Zustand entspricht.

Gemäß dem vorgeschlagenen Konzept lassen sich Probleme mit dem Lippenschluss und ein daraus folgendes Entweichen von Ausatemluft am Mundstück und damit am Messgerät vorbei ohne Weiteres vermeiden. Besonders für die Messung von Spurengasen kann ein Anwender des vorgeschlagenen Mundstücks durch die flötenähnliche Form intuitiv kontrolliert und nicht mit maximaler Ausatemfähigkeit atmen.

Es wird ein Mundstück für eine Vorrichtung zur Messung eines Parameters von Atemluft vorgestellt, wobei eine Endkante einer Wandung des Mundstücks eine Öffnung zum Einblasen von Atemluft in das Mundstück begrenzt, wobei sich eine Öffnungsfläche der Öffnung quer zu einer Strömungsachse von in das Mundstück einzublasender Atemluft erstreckt, dadurch gekennzeichnet, dass eine Kontur in Richtung der Strömungsachse gesehen der Öffnung eine D-Form oder eine Linsenform ausbildet und die Öffnung durch einen ersten Öffnungsabschnitt und einen an den ersten Öffnungsabschnitt angrenzenden zweiten Öffnungsabschnitt begrenzt ist.

Mit der Parametermessung der Atemluft mittels der Vorrichtung kann beispielsweise eine Lungenfunktion oder einer Alkoholgehalt im Blut einer zu untersuchenden Person ermittelt werden. Das Mundstück dient zum Einblasen der Atemluft in die Vorrichtung durch die zu untersuchende Person und wird dazu von der Person möglichst dicht mit den Lippen umschlossen. Bei der Wandung kann es sich um eine geschlossene Rundwand des Mundstücks handeln. Zwischen der die Öffnung bildenden Endkante und einer der Endkante gegenüberliegenden weiteren Endkante kann die Wandung insbesondere massiv und ohne zusätzliche Öffnungen verlaufen. Grob betrachtet kann die Wandung eine konische Form aufweisen, deren schmales Ende von der Öffnung gebildet wird. Die Strömungsachse kann einen Strömungsweg von an der Öffnung in das Mundstück eingeblasener Atemluft durch das Mundstück oder einen Strömungsweg von durch das Mundstück und die Öffnung angesaugter Luft repräsentieren. Unter der D-Form bzw. Linsenform kann - von einer auf das Mundstück blickenden Person aus betrachtet - eine Form eines liegenden Großbuchstabens D bzw. eine Form einer auf einer ihrer Hauptseiten liegenden Linse verstanden werden. Sowohl die D- als auch die Linsenform der Öffnungskontur können dadurch charakterisiert sein, dass eine erste Ansatzstelle, an der ein erstes Ende des ersten Öffnungsabschnitts und ein erstes Ende des zweiten Öffnungsabschnitts aneinander ansetzen, einen diskreten ersten Eckpunkt der Öffnungskontur ausbildet, und eine zweite Ansatzstelle, an der ein zweites Ende des ersten Öffnungsabschnitts und ein zweites Ende des zweiten Öffnungsabschnitts aneinander ansetzen, einen diskreten zweiten Eckpunkt der Öffnungskontur ausbildet. Der erste und der zweite Eckpunkt können einander spiegelsymmetrisch gegenüberliegen.

Gemäß einer Ausführungsform kann die Wandung einen zum Einblasen der Atemluft in die Öffnung von Lippen einer Person zu umschließenden Lippenkontaktbereich aufweisen. Der Lippenkontaktbereich kann sich aus einem an den ersten Öffnungsabschnitt angrenzenden und von einer Oberlippe der Person in Eingriff zu nehmenden Oberlippenabschnitt und einem an den zweiten Öffnungsabschnitt angrenzenden und von einer Unterlippe der Person in Eingriff zu nehmenden Unterlippenabschnitt zusammensetzen. Dabei kann der Oberlippenabschnitt eine Wölbung um die Strömungsachse aufweisen. In dieser Ausführung kann das Mundstück besonders gut an eine grundlegende Form der menschlichen Oberlippe angepasst werden.

Gemäß einer Ausführungsform kann der erste Öffnungsabschnitt sich von der Öffnung in Richtung der Strömungsachse weg erstrecken und in Richtung der Strömungsachse gesehen eine Form eines Bogens aufweisen, insbesondere wobei eine Krümmung des Bogens mit zunehmendem Abstand zur Öffnung geringer wird. Hierdurch wird eine turbulenzarme Luftströmung in dem Mundstück sichergestellt, die ein Einblasen von Luft mit einem sehr geringen Luftwiderstand ermöglicht, sodass die zu messenden Parameter der Atemluft sehr genau erfasst werden können.

Gemäß eine weiteren Ausführungsform kann die Wandung sich von einer der Endkante gegenüberliegenden weiteren Endkante der Wandung zu der Öffnung hin verjüngend ausgeformt sein. Ein so bewirkter sich stetig vergrößernder Querschnitt des Mundstücks kann es dem Anwender ermöglichen, die individuell beste Position für die Durchführung des Atemmanövers zu finden.

Beispielsweise kann eine kürzeste Verbindungsgerade von einer der Endkante der Wandung gegenüberliegenden weiteren Endkante der Wandung zu einem äußersten Endpunkt des Oberlippenabschnitts länger sein als eine kürzeste Verbindungsgerade von der weiteren Endkante des Mundstücks zu einem äußersten Endpunkt des Unterlippenabschnitts. Die Ausformung der Öffnung mit leicht vorstehendem Oberlippenabschnitt kann eine möglichst effektive Einleitung der Atemluft in das Mundstück gewährleisten.

Weiterhin kann das Mundstück an einer der Öffnung gegenüberliegenden Seite einen Auslassstutzen zum Auslassen der Atemluft aus dem Mundstück in eine mit dem Mundstück gekoppelte Vorrichtung aufweisen. Der Auslassstutzen kann beispielsweise einen ovalen Querschnitt aufweisen. Der Auslassstutzen - insbesondere in der Ausführung mit ovalem Querschnitt - weist den Vorteil einer verlustfreien und strömungsoptimalen Weiterleitung der Atemluft aus dem Mundstück in die Vorrichtung auf.

Gemäß einer Ausführungsform des Mundstücks kann eine Öffnungsfläche des Auslassstutzens größer als die Öffnungsfläche der Öffnung zum Einblasen von Atemluft in das Mundstück sein. So kann ohne Weiteres ein geeignet kontrolliertes Einblasen von Atemluft in das Mundstück erzielt werden.

Auch können sich eine Öffnungsfläche des Auslassstutzens und die Öffnungsfläche der Öffnung zum Einblasen von Atemluft in das Mundstück lediglich teilweise überlappen. Mit dieser Ausführungsform kann ein vorteilhafter Strömungsfluss der Atemluft durch das Mundstück gewährleistet werden. Die umlaufende Erhöhung gibt eine zusätzliche haptische Rückmeldung an den Lippen, damit Lippenkontakt mit dem Gerät vermieden wird. Diese könnte je nach Produktdesign auch höher ausgeführt werden.

Günstig ist es auch, wenn das Mundstück einen zwischen der Wandung und dem Anschlussstutzen angeordneten Flanschabschnitt zum Anflanschen des Mundstücks an eine Vorrichtung aufweist. So kann zum einen eine sichere und robuste Befestigung des Mundstücks an der Vorrichtung gewährleistet werden und zum anderen ein zu tiefes Einführen des Mundstücks in einen Mund eines Anwenders vermieden werden.

Gemäß einer besonderen Ausführungsform des Mundstücks kann der zweite Öffnungsabschnitt der Öffnung eine Form einer Geraden aufweisen oder geradlinig ausgeformt sein. Auch auf diese Weise kann eine möglichst effektive Einleitung der Atemluft in das Mundstück gewährleistet werden.

Insbesondere kann dabei der Unterlippenabschnitt des Lippenkontaktbereichs eine Form eines Mantelflächenabschnitts eines Zylinders aufweisen. Diese spezielle Ausprägung des Unterlippenabschnitts kann das Andrücken der Unterlippe eines Anwenders an das Mundstück erleichtern, sodass der dichte Lippenschluss um das Mundstück leichter gelingen kann.

Von einem Inneren des Mundstücks aus betrachtet, kann der Unterlippenabschnitt dabei eine konvexe Wölbung aufweisen. Auch mit dieser Ausführungsform kann ein Strömungsweg der Atemluft durch das Mundstück möglichst optimal gestaltet werden.

Gemäß einer weiteren besonderen Ausführungsform kann der zweite Öffnungsabschnitt der Öffnung eine Form eines weiteren Bogens aufweisen. Ein so ausgefertigtes Mundstück ist kostengünstig fertigbar und kann besonders vielseitig eingesetzt werden.

Es wird ein Atemluftmessgerät mit folgenden Merkmalen vorgestellt:
einer Vorrichtung zur Messung eines Parameters von Atemluft; und
einem Mundstück gemäß einer hier vorgestellten Variante; wobei das Mundstück über eine Atemluftschnittstelle mit der Vorrichtung gekoppelt oder koppelbar ist.

Speziell kann das Atemluftmessgerät als Steuergerät zur Steuerung eines Speils, beispielsweise eines Computerspiels ausgestaltet sein.

Auch durch diese Ausführungsvariante der Erfindung in Form einer Vorrichtung kann die der Erfindung zugrunde liegende Aufgabe schnell und effizient gelöst werden. Die Vorrichtung kann beispielsweise im medizinischen Bereich zur Atemgasmessung eingesetzt werden. In einer besonderen Ausführung kann die Vorrichtung auch zur atemgesteuerten Bedienung von Computern verwendet werden, z. B. für körperlich behinderte Menschen oder zur Steuerung von (Computer-) Spielen.

Der hier vorgestellte Ansatz wird nachstehend anhand der beigefügten Zeichnungen beispielhaft näher erläutert. Es zeigen:
Fig. 1 eine schematische Darstellung einer Vorrichtung zur Messung eines Parameters von Atemluft, gemäß einem Ausführungsbeispiel der vorliegenden Erfindung;
Fig. 2 bis 7 verschiedene Ansichten eines Mundstücks für eine Vorrichtung zur Messung eines Parameters von Atemluft, gemäß einem Ausführungsbeispiel der vorliegenden Erfindung; und
Fig. 8 eine Vorderansicht eines Mundstücks für eine Vorrichtung zur Messung eines Parameters von Atemluft, gemäß einem weiteren Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

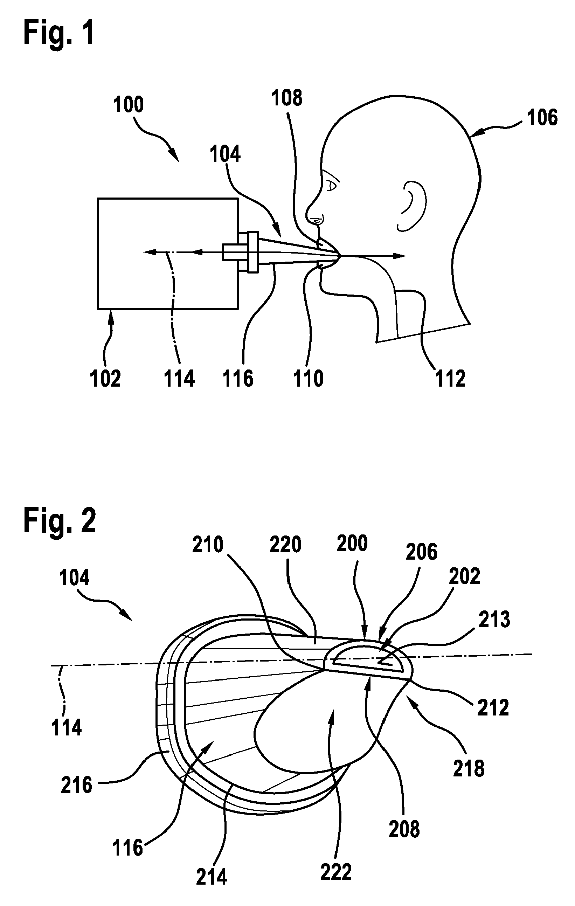

Fig. 1 zeigt eine schematische Darstellung eines Atemluftmessgeräts 100 gemäß einem Ausführungsbeispiel der vorliegenden Erfindung. Bei dem Atemluftmessgerät 100 handelt es sich im gezeigten Ausführungsbeispiel um einen Apparat oder einen Teil eines Apparats zum Durchführen eines Lungenfunktionstests. Das Atemluftmessgerät 100 weist eine Vorrichtung 102 zur Messung eines Parameters von Atemluft sowie ein mit der Vorrichtung 102 gekoppeltes Mundstück 104 auf.

Bei einem Einsatz des Atemluftmessgeräts 100 nimmt ein Anwender 106, hier eine zu untersuchende Person, einen eine Öffnung aufweisenden Endbereich des Mundstücks 104 in den Mund und bildet einen möglichst dichten Lippenschluss um das Mundstück 104 durch Pressen der Oberlippe 108 an eine Oberseite des Mundstücks 104 und Pressen der Unterlippe 110 an eine Unterseite des Mundstücks 104. Mit einem anschließenden Ausatmen des Anwenders 106 strömt Atemluft 112 aus der Lunge des Anwenders entlang einer Strömungsachse 114 des Mundstücks 104 durch das Mundstück 104 und in die mit dem Mundstück 104 gekoppelte Vorrichtung 102. Dort wird die Atemluft 112 bzw. ein Druck der Atemluft 112 analysiert.

Wie die Darstellung in Fig. 1 zeigt, ist eine Wandung 116 des Mundstücks 104 sich verjüngend ausgebildet, wobei ein schmales Ende der Wandung 116 einen von den Lippen 108, 110 zu umschließenden Kontaktbereich des Mundstücks 104 bildet. Das Mundstück 104 weist bei dem in Fig. 1 gezeigten Ausführungsbeispiel im Querschnitt eine Linsenform auf, auf die weiter unten noch detaillierter eingegangen wird.

Bei einer mithilfe des Mundstücks 104 durchgeführten Atemgasdiagnose muss zur Messung der Gaszusammensetzung der Atemluft der gesamte Atemfluss 112 dem Messgerät 100 zugeführt werden. Bei der Messung der Lungenfunktion und der Hauptbestandteile der Atemluft wird mit hohen Flussmengen geatmet, bei der Messung von Spurenbestandteilen wie z. B. Alkohol oder NO werden niedrige Atemflüsse 112 gemessen.

Fig. 2 zeigt in einer perspektivischen Darstellung ein weiteres Ausführungsbeispiel des Mundstücks 104 für eine Vorrichtung zur Messung eines Parameters von Atemluft. Die Wandung 116 hat einen grob konunsförmigen Verlauf. An einer dem Anwender zuzuwendenden Seite des Mundstücks 104, dem schmalen Ende des Konus, formt eine Endkante 200 der Wandung 116 eine Öffnung 202 zum Einblasen von Atemluft in das Mundstück 104.

Bei dem in Fig. 2 gezeigten beispielhaften Mundstück 104 weist eine Kontur der Öffnung 202 eine Form eines liegenden Großbuchstabens D auf. Entsprechend weist ein erster Öffnungsabschnitt 206 der Öffnung 202 eine Form eines Bogens und ein zweiter Öffnungsabschnitt 208 der Öffnung 202 eine Form einer Geraden auf.

Wie die Darstellung in Fig. 2 zeigt, bildet die Öffnung 202 zwei einander spiegelsymmetrisch gegenüberliegende Eckpunkte aus. Ein erster Eckpunkt 210 wird an einer ersten Ansatzstelle gebildet, an der ein erstes Ende des ersten Öffnungsabschnitts 206 und ein erstes Ende des zweiten Öffnungsabschnitts 208 aneinander ansetzen. Ein zweiter Eckpunkt 212 wird an einer zweiten Ansatzstelle gebildet, an der ein zweites Ende des ersten Öffnungsabschnitts 206 und ein zweites Ende des zweiten Öffnungsabschnitts 208 aneinander ansetzen. Eine Öffnungsfläche 213 der Öffnung 202 erstreckt sich quer zu der mittels einer Strichlinie in der Darstellung gekennzeichneten Strömungsachse 114 der Ausatem- bzw. Einatemluft durch das Mundstück 104.

Eine der Endkante 200 der Wandung 116 gegenüberliegende weitere Endkante 214 der Wandung 116 weist einen maximalen Durchmesser des konusförmigen Mundstücks 104 auf. Die weitere Endkante 214 bildet einen vollumfänglichen Flansch 216 zum Ansetzen des Mundstücks 104 an einer Vorrichtung, beispielsweise dem in Fig. 1 gezeigten Atemmessgerät, an. Der Flansch 216 kann gemäß Ausführungsbeispielen auch separat hergestellt und im Fertigungsprozess des Mundstücks 104 mit der weiteren Endkante 214 fest verbunden worden sein.

Ein an die Öffnung 202 angrenzender Bereich der Wandung 116 bildet einen Lippenkontaktbereich 218 aus, der beim Einsatz des Mundstücks 104 zum Einblasen der Atemluft in die Öffnung 202 bzw. zum Saugen von Luft aus der Öffnung 202 von dem Anwender mit den Lippen umschlossen wird. Der Lippenkontaktbereich 218 setzt sich aus einem an den ersten Öffnungsabschnitt 206 angrenzenden Oberlippenabschnitt 220 und einem an den zweiten Öffnungsabschnitt 208 angrenzenden Unterlippenabschnitt 222 zusammen.

Der Oberlippenabschnitt 220 weist eine Wölbung um die Strömungsachse 114 auf. Der Unterlippenabschnitt 222 schließt eine Ausnehmung aus der konusförmigen Wandung 116 und wölbt sich in Richtung eines Inneren des Mundstücks 104 zwischen der Endkante 200 der Wandung 116 mit minimalem Durchmesser und einer Position im Mittelbereich der Wandung 116 mit wesentlich größerem, hier jedoch nicht maximalen, Durchmesser.

Im Gebrauch, wenn das Mundstück 104 mit einer Vorrichtung zur Messung eines Parameters von Atemluft. gekoppelt ist, bildet der Anwender zum Einblasen von Luft in das Mundstück 104 bzw. zum Saugen von Luft aus dem Mundstück 104 einen Lippenschluss um den Lippenkontaktbereich 218, indem der Anwender die Oberlippe auf den Oberlippenabschnitt 220 presst und die Unterlippe auf den Unterlippenabschnitt 222 presst.

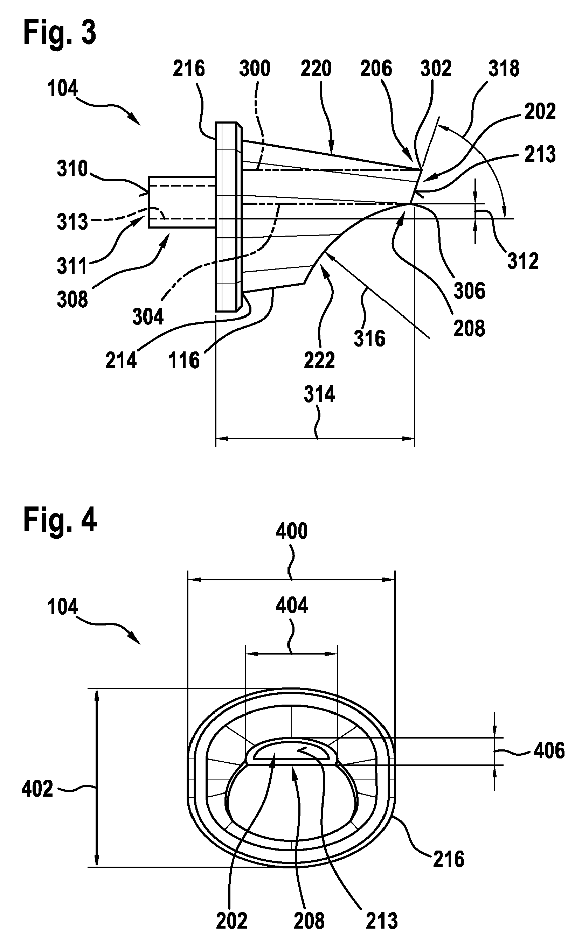

Fig. 3 zeigt das beispielhafte Mundstück 104 aus Fig. 2 in einer Seitenansicht. Hier ist gut der sich von weiteren Endkante 214 der Wandung 116 zu der Öffnung 202 hin verjüngende Verlauf der Wandung 116 zu erkennen. Auch ist in der Seitenansicht zu sehen, dass der erste Öffnungsabschnitt 206 der Öffnung 202 gegenüber dem zweiten Öffnungsabschnitt 208 vorsteht. Entsprechend ist eine kürzeste Verbindungsgerade 300 zwischen der weiteren Endkante 214 der Wandung 116 zu einem äußersten Endpunkt 302 des Oberlippenabschnitts 220 größer ist als eine kürzeste Verbindungsgerade 304 von der weiteren Endkante 214 der Wandung 116 des Mundstücks 104 zu einem äußersten Endpunkt 306 des Unterlippenabschnitts 222.

In der in Fig. 3 dargestellten Seitenansicht des beispielhaften Mundstücks 104 formt die den Unterlippenabschnitt 222 bildende Ausnehmung aus der konusförmigen Wandung 116 eine Form eines Mantelflächenabschnitts eines Zylinders. Der Unterlippenabschnitt 222 beginnt direkt an dem zweiten Öffnungsabschnitt 208 und endet beabstandet von der weiteren Endkante 214 der Wandung 116. Von einem Inneren des Mundstücks 104 aus betrachtet, weist der Unterlippenabschnitt 222 bei dem in Fig. 3 gezeigten Ausführungsbeispiel eine konvexe Wölbung auf.

Die Seitenansicht des beispielhaften Mundstücks 104 in Fig. 3 zeigt einen Auslassstutzen 308 zum Auslassen von Ausatemluft aus dem Mundstück 104 in eine mit dem Mundstück 104 gekoppelte Vorrichtung. Der Auslassstutzen 308 setzt an einer der Öffnung 202 gegenüberliegenden Seite des Mundstücks 104 an dem Flansch 216 an und weist hier eine Form eines Rohres mit gleichbleibendem Durchmesser auf.

Aus der Darstellung in Fig. 3 ist ersichtlich, dass sich eine Öffnungsfläche 310 einer Öffnung 311 des Auslassstutzens 308 und die Öffnungsfläche 213 der Öffnung 202 nicht vollständig überlappen. Im gezeigten Ausführungsbeispiel liegt die Öffnungsfläche 310 des Auslassstutzens 308 tiefer als die Öffnungsfläche 213 der Öffnung 202. Konkret beträgt ein Versatz 312 zwischen einer Außenkante des zweiten Öffnungsabschnitts 208 und einer Oberfläche einer Innenwand 313 des Auslassstutzens 308 3 Millimeter.

Als weitere konkrete Maße beträgt eine Länge 314 des Mundstücks 104 von einer dem Anschlussstutzen 308 zugewandten Hauptseite des Flansches 216 zu dem Endpunkt 306 des zweiten Öffnungsabschnitts 208 43 Millimeter. Ein Radius 316 des einen Abschnitt einer Zylinderwand bildenden Unterlippenabschnitts 222 beträgt 30 Millimeter, und ein Winkel 318 zwischen dem Oberlippenabschnitt 220 und einer Erstreckung der Innenwand 313 des Anschlussstutzens 308 beträgt 75 Grad.

Fig. 4 zeigt eine Vorderansicht des beispielhaften Mundstücks 104 aus Fig. 2. Eine Gesamtbreite 400 des Mundstücks 104 inklusive des Flansches 216 beträgt bei dem beispielhaften Mundstück 104 47 Millimeter und eine Gesamthöhe 402 inklusive des Flansches 216 41 Millimeter. Eine maximale Breite 404 der D-förmigen Öffnung 202, die bei dem gezeigten Ausführungsbeispiel der Länge des geraden zweiten Öffnungsabschnitts 208 entspricht, beträgt 22 Millimeter und eine maximale Höhe 406 der Öffnung 202 7 Millimeter.

In der Darstellung in Fig. 4 ist gut zu erkennen, wie der sich stetig vergrößernde Querschnitt des Mundstücks 104 es dem Anwender ermöglicht, die individuell beste Position für die Durchführung des Atemmanövers zu finden, ohne dass am Gerät vorbei geblasen wird.

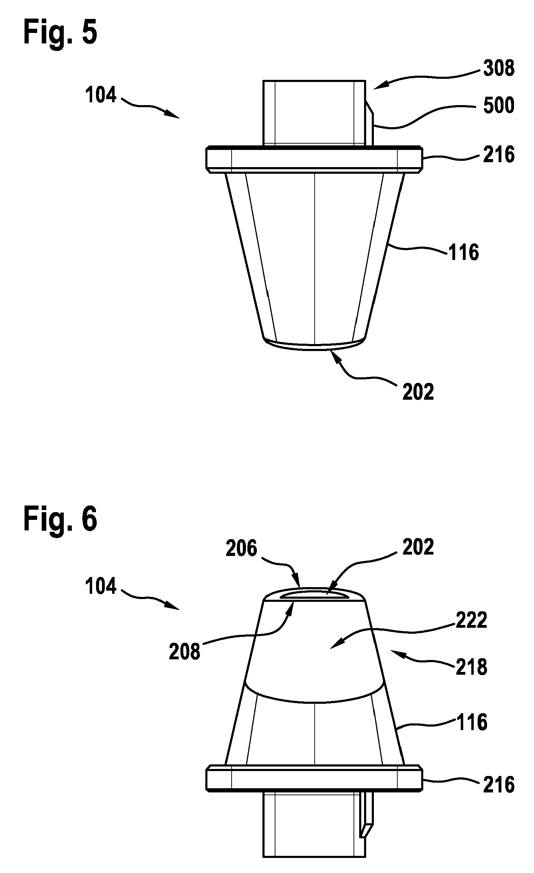

Fig. 5 zeigt eine Draufsicht auf das beispielhafte Mundstück 104 aus Fig. 2. Hier ist nochmals der sich verjüngende Verlauf der Wandung 116 des Mundstücks 104 von dem Flansch 216 zu der Öffnung 202 gut zu erkennen. Auch zeigt sich, dass die Wandung 116 zwischen dem Flansch 216 und der Öffnung 202 massiv ausgebildet ist, also keinerlei Öffnungen aufweist.

Der in Fig. 5 gezeigte beispielhafte Anschlussstutzen 308 ist seitlich mit einem Vorsprung 500 ausgestattet. Der Vorsprung 500 erlaubt ein korrekt positioniertes Einsetzen des Mundstücks 104 in einen Aufnahmebereich einer mit dem Mundstück 104 zu koppelnden Vorrichtung zur Messung eines Parameters von Atemluft und gemäß einem Ausführungsbeispiel zum sicheren Einrasten des Mundstücks 104 in die Vorrichtung. Außerdem kann der Vorsprung 500 zur Detektion eines korrekt aufgesteckten Mundstücks in der Messvorrichtung dienen.

Fig. 6 zeigt eine Untenansicht des beispielhaften Mundstücks 104 aus Fig. 2. Hier ist gut zu erkennen, dass der sich von der Öffnung 202 in Richtung des Flansches 216 graduell verbreiternde Unterlippenabschnitt 222 des Lippenkontaktbereichs 218 sich über mehr als die Hälfte einer Unterseite der Wandung 116 erstreckt. So kann jeder Anwender des Mundstücks 104 für einen optimalen Lippenschluss um den Lippenkontaktbereich 218 komfortabel die geeignete Position für die Unterlippe finden. Auch zeigt sich in der Darstellung in Fig. 6 gut der Vorstand des ersten Öffnungsabschnitts 206 der Öffnung 202 gegenüber dem zweiten Öffnungsabschnitt 208.

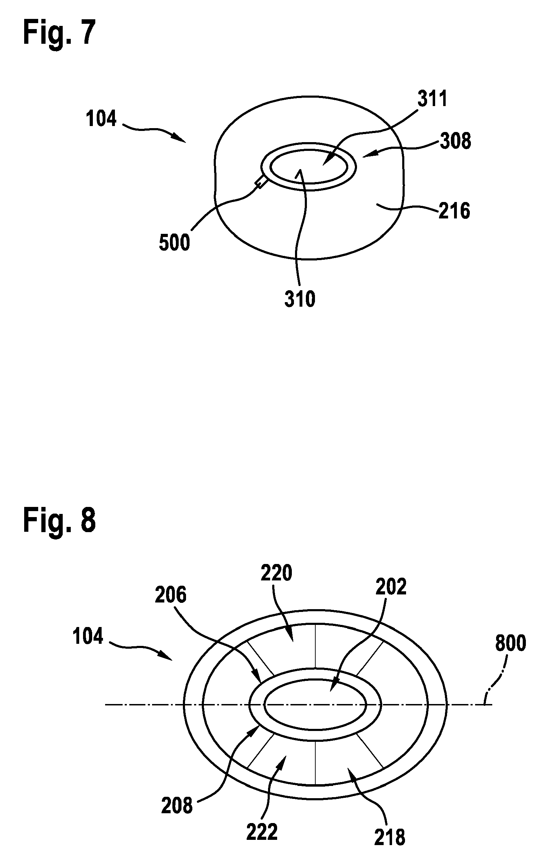

Fig. 7 zeigt eine Rückansicht des beispielhaften Mundstücks 104 aus Fig. 2. Hier präsentiert sich eine von einem Anwender des Mundstücks 104 im Einsatz abgewandte Hauptseite des Flansches 216, aus deren ungefährer Mitte der Anschlussstutzen 308 ragt. Bei dem in Fig. 7 gezeigten Ausführungsbeispiel weist eine Kontur des Anschlussstutzens 308 im Querschnitt eine Form eines symmetrischen, lang gezogenen, liegenden Ovals auf. Damit korrespondiert die Form des Anschlussstutzens 308 teilweise mit der Form der Öffnung 202 am anderen Ende des Mundstücks 104. Ein Vergleich der Darstellung in Fig. 7 mit der Darstellung in Fig. 4 zeigt, dass bei dem beispielhaften Mundstück 104 die Öffnungsfläche 310 der Öffnung 311 des Auslassstutzens 308 größer als die Öffnungsfläche 213 der Öffnung 202 zum Einblasen von Atemluft in das Mundstück 104 ist.

Fig. 8 zeigt eine Vorderansicht des beispielhaften Mundstücks 104 aus Fig. 1. In der Darstellung ist zu erkennen, dass die Öffnung 202 zum Einblasen von Atemluft in das Mundstück 104 eine Linsenform aufweist. Hier haben sowohl der erste Öffnungsabschnitt 206 als auch der zweite Öffnungsabschnitt 208 der Öffnung 202 die Bogenform, die bei dem in den Figuren 2 bis 7 vorgestellten Ausführungsbeispiel des Mundstücks 104 nur der erste Öffnungsabschnitt 206 aufweist. Bei dem in Fig. 8 gezeigten Ausführungsbeispiel des Mundstücks 104 sind der Oberlippenabschnitt 220 und der Unterlippenabschnitt 222 des Lippenkontaktbereichs 218 bezüglicher einer Spiegelungsachse 800 spiegelsymmetrisch.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Mundstück (104) für eine Vorrichtung (102) zur Messung eines Parameters von Atemluft (112), wobei eine Endkante (200) einer Wandung (116) des Mundstücks (104) eine Öffnung (202) zum Einblasen von Atemluft (112) in das Mundstück (104) begrenzt, wobei sich eine Öffnungsfläche (213) der Öffnung (202) quer zu einer Strömungsachse (114) von in das Mundstück (104) einzublasender Atemluft (112) erstreckt, **dadurch gekennzeichnet, dass** eine Kontur der Öffnung (202) in Richtung der Strömungsachse gesehen eine D-Form oder eine Linsenform ausbildet und die Öffnung (202) durch einen ersten Öffnungsabschnitt (206) und einen an den ersten Öffnungsabschnitt (206) angrenzenden zweiten Öffnungsabschnitt (208) begrenzt ist

2. Mundstück (104) gemäß Anspruch 1, **dadurch gekennzeichnet, dass** die Wandung (116) einen zum Einblasen der Atemluft (112) in die Öffnung (202) von Lippen einer Person (106) zu umschließenden Lippenkontaktbereich (218) aufweist, der sich aus einem an den ersten Öffnungsabschnitt (206) angrenzenden und von einer Oberlippe (108) der Person (106) in Eingriff zu nehmenden Oberlippenabschnitt (220) und einem an den zweiten Öffnungsabschnitt (208) angrenzenden und von einer Unterlippe (110) der Person (106) in Eingriff zu nehmenden Unterlippenabschnitt (222) zusammensetzt, wobei der Oberlippenabschnitt (220) eine Wölbung um die Strömungsachse (114) aufweist.

3. Mundstück (104) gemäß einem der vorangegangenen Ansprüche, **dadurch gekennzeichnet, dass** der erste Öffnungsabschnitt (206) sich von der Öffnung in Richtung der Strömungsachse weg erstreckt und in Richtung der Strömungsachse gesehen eine Form eines Bogens aufweist, insbesondere wobei eine Krümmung des Bogens mit zunehmendem Abstand zur Öffnung geringer wird.

4. Mundstück (104) gemäß einem der vorangegangenen Ansprüche, **dadurch gekennzeichnet, dass** die Wandung (116) sich von einer der Endkante (200) gegenüberliegenden weiteren Endkante (214) der Wandung (116) zu der Öffnung (202) hin verjüngend ausgeformt ist.

5. Mundstück (104) gemäß einem der vorangegangenen Ansprüche, **dadurch gekennzeichnet, dass** eine kürzeste Verbindungsgerade (300) von einer der Endkante (200) der Wandung (116) gegenüberliegenden weiteren Endkante (214) der Wandung (116) zu einem äußersten Endpunkt (302) des Oberlippenabschnitts (220) länger ist als eine kürzeste Verbindungsgerade (304) von der weiteren Endkante (214) des Mundstücks (104) zu einem äußersten Endpunkt (306) des Unterlippenabschnitts (222).

6. Mundstück (104) gemäß einem der vorangegangenen Ansprüche, **dadurch gekennzeichnet, dass** das Mundstück (104) an einer der Öffnung (202) gegenüberliegenden Seite einen Auslassstutzen (308) zum Auslassen der Atemluft (112) aus dem Mundstück (104) in eine mit dem Mundstück (104) gekoppelte Vorrichtung (102) aufweist, wobei der Auslassstutzen (308) einen ovalen Querschnitt aufweist.

7. Mundstück (104) gemäß Anspruch 6, **dadurch gekennzeichnet, dass** eine Öffnungsfläche (310) des Auslassstutzens (308) größer als die Öffnungsfläche (213) der Öffnung (202) zum Einblasen von Atemluft (112) in das Mundstück (104) ist.

8. Mundstück (104) gemäß einem der Ansprüche 6 oder 7, **dadurch gekennzeichnet, dass** sich eine Öffnungsfläche (310) des Auslassstutzens (308) und die Öffnungsfläche (213) der Öffnung (202) zum Einblasen von Atemluft (112) in das Mundstück (104) lediglich teilweise überlappen.

9. Mundstück (104) gemäß einem der Ansprüche 6 bis 8, **dadurch gekennzeichnet, dass** das Mundstück (104) einen zwischen der Wandung (116) und dem Anschlussstutzen angeordneten Flanschabschnitt (216) zum Anflanschen des Mundstücks (104) an eine Vorrichtung (102) aufweist.

10. Mundstück (104) gemäß einem der Ansprüche 2 bis 9, **dadurch gekennzeichnet, dass** der zweite Öffnungsabschnitt (208) der Öffnung (202) geradlinig ausgeformt ist.

11. Mundstück (104) gemäß einem der Ansprüche 2 bis 10, **dadurch gekennzeichnet, dass** der Unterlippenabschnitt (222) des Lippenkontaktbereichs (218) eine Form eines Mantelflächenabschnitts eines Zylinders aufweist.

12. Mundstück (104) gemäß einem der Ansprüche 2 bis 11, **dadurch gekennzeichnet, dass** der Unterlippenabschnitt (222) des Lippenkontaktbereichs (218) von einem Inneren des Mundstücks (104) aus betrachtet eine konvexe Wölbung aufweist.

13. Mundstück (104) gemäß einem der Ansprüche 2 bis 9, **dadurch gekennzeichnet, dass** der zweite Öffnungsabschnitt (208) der Öffnung (202) eine Form eines weiteren Bogens aufweist.

14. Atemluftmessgerät (100) oder Steuergerät für ein Spiel mit folgenden Merkmalen:
einer Vorrichtung (102) zur Messung eines Parameters von Atemluft (112); und
einem Mundstück (104) gemäß einem der vorangegangenen Ansprüche; wobei das Mundstück (104) über eine Atemluftschnittstelle mit der Vorrichtung (102) gekoppelt oder koppelbar ist.
